(19) Europäisches Patentamt / European Patent Office / Office européen des brevets

(11) **EP 3 340 990 B1**

(12) **EUROPEAN PATENT SPECIFICATION**

(45) Date of publication and mention of the grant of the patent:
**25.09.2019 Bulletin 2019/39**

(21) Application number: **16763585.3**

(22) Date of filing: **24.08.2016**

(51) Int Cl.:
***A61K 31/517*** *(2006.01)* ***A61K 31/519*** *(2006.01)*
***A61P 35/00*** *(2006.01)*

(86) International application number:
**PCT/IB2016/055044**

(87) International publication number:
**WO 2017/037576 (09.03.2017 Gazette 2017/10)**

(54) **PHARMACEUTICAL COMBINATIONS COMPRISING (A) THE CYCLIN DEPENDENT KINASE 4/6 (CDK4/6) INHIBITOR LEE011 (=RIBOCICLIB), AND (B) THE EPIDERMAL GROWTH FACTOR RECEPTOR (EGFR) INHIBITOR ERLOTINIB, FOR THE TREATMENT OR PREVENTION OF CANCER**

PHARMAZEUTISCHE KOMBINATIONEN MIT (A) INHIBITOR LEE011 (=RIBOCICLIB) DER CYCLIN-ABHÄNGIGEN KINASE 4/6 (CDK4/6) UND (B) INHIBITOR ERLOTINIB DES EPIDERMALEN WACHSTUMSFAKTORREZEPTOR (EGFR) ZUR BEHANDLUNG ODER VORBEUGUNG VON KREBS

COMBINAISONS PHARMACEUTIQUES COMPRENANT (A) L'INHIBITEUR DE KINASE DÉPENDANTE DE LA CYCLINE 4/6 (CDK4/6) LEE011 (=RIBOCICLIB), ET (B) L'INHIBITEUR DE RÉCEPTEUR DU FACTEUR DE CROISSANCE ÉPIDERMIQUE (EGFR) ERLOTINIB, POUR LE TRAITEMENT OU LA PRÉVENTION DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2015 US 201562211031 P**

(43) Date of publication of application:
**04.07.2018 Bulletin 2018/27**

(73) Proprietor: **Novartis AG**
**4056 Basel (CH)**

(72) Inventors:
- **CAPONIGRO, Giordano**
  **Cambridge, Massachusetts 02139 (US)**
- **HORN-SPIROHN, Thomas**
  **Cambridge, Massachusetts 02139 (US)**
- **LEHAR, Joseph**
  **Cambridge, Massachusetts 02139 (US)**

(74) Representative: **Rudge, Sewkian**
**Novartis AG**
**Patent Department**
**Lichtstrasse 35**
**4056 Basel (CH)**

(56) References cited:
- **Sadhna r. Vora ET AL: "CDK 4/6 Inhibitors Sensitize PIK3CA Mutant Breast Cancer to PI3K Inhibitors; supplementary information included", Cancer Cell, 1 July 2014 (2014-07-01), pages 136-149, XP055314327, DOI: 10.1016/j.ccr.2014.05.020 Retrieved from the Internet: URL:https://www.ncbi.nlm.nih.gov/pmc/articles/PMC4155598/pdf/nihms612211.pdf [retrieved on 2016-10-26]**
- **PHILIPPE DEPEILLE ET AL: "RasGRP1 opposes proliferative EGFR-SOS1-Ras signals and restricts intestinal epithelial cell growth", NATURE CELL BIOLOGY, vol. 17, no. 6, 1 April 2015 (2015-04-01), pages 804-815, XP055315911, GB ISSN: 1465-7392, DOI: 10.1038/ncb3175**
- **F. YAMASAKI ET AL: "Sensitivity of breast cancer cells to erlotinib depends on cyclin-dependent kinase 2 activity", MOLECULAR CANCER THERAPEUTICS, vol. 6, no. 8, 1 August 2007 (2007-08-01), pages 2168-2177, XP055315930, US ISSN: 1535-7163, DOI: 10.1158/1535-7163.MCT-06-0514**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 340 990 B1

- A. KATHLEEN MCCLENDON ET AL: "CDK4/6 inhibition antagonizes the cytotoxic response to anthracycline therapy", CELL CYCLE, vol. 11, no. 14, 15 January 2012 (2012-01-15), pages 2747-2755, XP055315974, US ISSN: 1538-4101, DOI: 10.4161/cc.21127

**Description**

TECHNICAL FIELD

The present disclosure relates to pharmaceutical combinations comprising (a) a cyclin dependent kinase 4/6 (CDK4/6) inhibitor compound, and (b) an epidermal growth factor receptor (EGFR) inhibitor, for the treatment or prevention of cancer. The disclosure also provides related pharmaceutical compositions, uses, and methods of treatment or prevention of cancer.

BACKGROUND

Tumor development is closely associated with genetic alteration and deregulation of cyclin dependent kinases (CDKs) and their regulators, suggesting that inhibitors of CDKs may be useful anti-cancer therapeutics. Indeed, early results suggest that transformed and normal cells differ in their requirement for, *e.g.*, cyclin D/CDK4/6 and that it may be possible to develop novel antineoplastic agents devoid of the general host toxicity observed with conventional cytotoxic and cytostatic drugs.

The function of CDKs is to phosphorylate and thus activate or deactivate certain proteins, including, e.g., retinoblastoma proteins, lamins, histone HI, and components of the mitotic spindle. The catalytic step mediated by CDKs involves a phospho-transfer reaction from ATP to the macromolecular enzyme substrate. Several groups of compounds (reviewed in, e.g., Fischer, P. M. Curr. Opin. Drug Discovery Dev. 2001, 4, 623-634) have been found to possess antiproliferative properties by virtue of CDK-specific ATP antagonism.

At a molecular level, mediation of CDK/cyclin complex activity requires a series of stimulatory and inhibitory phosphorylation, or dephosphorylation, events. CDK phosphorylation is performed by a group of CDK activating kinases (CAKs) and/or kinases such as weel, Myt1 and Mik1. Dephosphorylation is performed by phosphatases such as Cdc25(a & c), PP2A, or KAP.

CDK/cyclin complex activity may be further regulated by two families of endogenous cellular proteinaceous inhibitors: the Kip/Cip family, or the INK family. The INK proteins specifically bind CDK4 and CDK6. $p16^{ink4}$ (also known as MTS1) is a potential tumor suppressor gene that is mutated or deleted in a large number of primary cancers. The Kip/Cip family contains proteins such as $p21^{Cip1,Waf1}$, $p27^{Kip1}$ and $p57^{kip2}$, where p21 is induced by p53 and is able to inactivate the CDK2/cyclin(E/A) complex. Atypically low levels of p27 expression have been observed in breast, colon and prostate cancers. Conversely, over-expression of cyclin E in solid tumors has been shown to correlate with poor patient prognosis. Over-expression of cyclin D1 has been associated with esophageal, breast, squamous, and non-small cell lung carcinomas.

The pivotal roles of CDKs, and their associated proteins, in coordinating and driving the cell cycle in proliferating cells have been outlined above. Some of the biochemical pathways in which CDKs play a key role have also been described. The development of monotherapies for the treatment of proliferative disorders, such as cancers, using therapeutics targeted generically at CDKs, or at specific CDKs, is therefore potentially highly desirable.

The Epidermal Growth Factor Receptor (EGFR, aka ErbB-1; HER1 in humans), is a receptor for ligands of the epidermal growth factor family. Several types of cancers are known to be dependent on EGFR over-activity or over-expression, such as lung cancer, anal cancers, glioblastoma multiforme and many other mainly epithelial cancers.

Cancer is often dependent on the genetic alteration of receptor tyrosine kinases (RTKs) e.g. by point mutation, gene amplification or chromosomal translocation which leads to uncontrolled activity of these RTKs which thus become oncogenic. Cell proliferation of cancer cells is dependent on the activity of these aberrant RTKs.

When treating the resulting proliferative diseases, often inhibitors of the oncogene RTK involved are used. However, often, after a certain time of treatment, resistance to the drug used is observed. One mechanism of resistance can involve the target RTK, compromising binding or activity of the therapeutic agent. Another mechanism is compensatory activation of an alternative kinase that continues to drive cancer growth when the primary kinase is inhibited. A well-characterized example covering both types of mechanisms is acquired resistance to the epidermal growth factor receptor (EGFR) gefitinib and erlotinib in non-small cancer (NSCLC) carrying activating EGFR mutations (see Lynch, T. J., et al.,. N Engl J Med, 350: 2129-2139, 2004; or Paez, J. G., et al., Science, 304: 1497-1500, 2004). For example, MET activation can compensate for loss of EGFR activity (by inhibition) by downstream activation of signal molecules such as HER3, such as MET amplification may compensate, or its ligand hepatocyte growth factor may activate MET (see Engelman, J. A., et al., Science, 316: 1039-1043, 2007; Yano, S., et al., Cancer Res, 68: 9479-9487, 2008; and Turke, A. B., et al., Cancer Cell, 17: 77-88, 2010). It is also known that MET-dependent cancer cell lines (the proliferation of which depends on the activity of MET) can be rescued from MET inhibitors by ligand-induced EGFR activation (see Bachleitner-Hofmann, T., et al., Mol Cancer Ther, 7: 3499-3508, 2008).

In spite of numerous treatment options for cancer patients, there remains a need for effective and safe therapeutic agents and a need for their preferential use in combination therapy. In particular, there is a need for effective methods

of treating cancers, especially those cancers that have been resistant and/or refractive to current therapies.

SUMMARY

In a first aspect, provided herein is a pharmaceutical combination comprising:

(a) a first compound having the structure of formula (I):

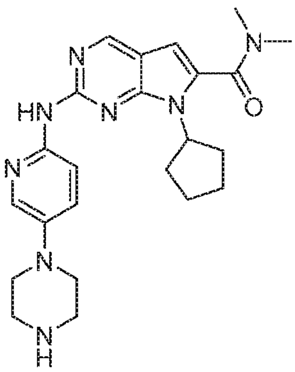

(I)

or a pharmaceutically acceptable salt or solvate thereof, and
(b) a second compound having the structure of formula (II):

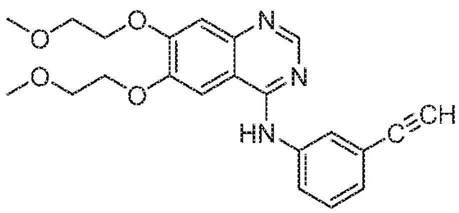

(II)

or a pharmaceutically acceptable salt or solvate thereof.

In an embodiment, the compound having the structure of formula (I), or a pharmaceutically acceptable salt or solvate thereof, and the compound having the structure of formula (II), or a pharmaceutically acceptable salt or solvate thereof, are in the same formulation.

In an embodiment, the compound having the structure of formula (I), or a pharmaceutically acceptable salt or solvate thereof, and the compound having the structure of formula (II), or a pharmaceutically acceptable salt or solvate thereof, are in separate formulations.

In an embodiment, the combination of the first aspect is for simultaneous or sequential administration.

In a particular embodiment of the pharmaceutical combinations described *supra,* the first compound is the succinate salt of the compound having the structure of formula (I).

In a second aspect, provided herein is a method for the treatment or prevention of cancer in a subject in need thereof, comprising administering to the subject a therapeutically effective amount of a pharmaceutical combination according to any one of the embodiments described *supra.*

In an embodiment, the cancer is selected from the group consisting of melanoma, lung cancer (including non-small-cell lung cancer (NSCLC)), colorectal cancer (CRC), breast cancer, kidney cancer, renal cell carcinoma (RCC), liver cancer, acute myelogenous leukemia (AML), myelodysplastic syndromes (MDS), thyroid cancer, pancreatic cancer, neurofibromatosis and hepatocellular carcinoma.

In a particular embodiment, the cancer is colorectal cancer.

In a third aspect, provided herein is a pharmaceutical combination as described *supra* for use in the treatment or prevention of cancer.

In a fourth aspect, provided herein is a pharmaceutical combination as described *supra* for use in the manufacture

of a medicament for the treatment or prevention of cancer.

In certain embodiments of the third and fourth aspects, the cancer is selected from the group consisting of melanoma, lung cancer (including non-small-cell lung cancer (NSCLC)), colorectal cancer (CRC), breast cancer, kidney cancer, renal cell carcinoma (RCC), liver cancer, acute myelogenous leukemia (AML), myelodysplastic syndromes (MDS), thyroid cancer, pancreatic cancer, neurofibromatosis and hepatocellular carcinoma.

In a particular embodiment, the cancer is colorectal cancer.

In a fifth aspect, provided herein is the use of a pharmaceutical combination as described *supra* for the manufacture of a medicament for the treatment or prevention of cancer.

In a sixth aspect, provided herein is the use of a pharmaceutical combination as described *supra* for the treatment or prevention of cancer.

In particular embodiments of the fifth and sixth aspects, the cancer is selected from the group consisting of melanoma, lung cancer (including non-small-cell lung cancer (NSCLC)), colorectal cancer (CRC), breast cancer, kidney cancer, renal cell carcinoma (RCC), liver cancer, acute myelogenous leukemia (AML), myelodysplastic syndromes (MDS), thyroid cancer, pancreatic cancer, neurofibromatosis and hepatocellular carcinoma.

In a particular embodiment, the cancer is colorectal cancer.

In a seventh aspect, provided herein is a pharmaceutical composition comprising:

(a) a first compound having the structure of formula (I):

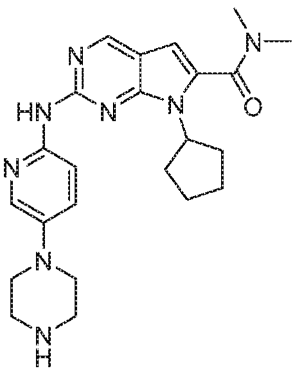

(I)

or a pharmaceutically acceptable salt or solvate thereof, and

(b) a second compound having the structure of formula (II):

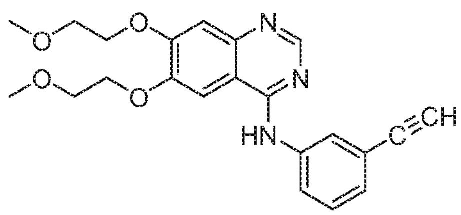

(II)

or a pharmaceutically acceptable salt or solvate thereof.

In an embodiment, the pharmaceutical composition comprises one or more excipients.

BRIEF DESCRIPTION OF THE FIGURES

**Figure 1** shows dose-response curves for LEE011 and erlotinib and the combination of LEE011 and erlotinib over 15 colorectal cancer cell lines. The x-axis indicates the log10 of the treatment dilution; the y-axis indicates the cell count after treatment relative to DMSO. The strong dashed line indicates the number of cells before the start of the

treatment ('baseline').

**Figure 2** shows maximum Caspase 3/7 induction for LEE011 and erlotinib and the combination of LEE011 and erlotinib in 15 colorectal cancer cell lines and after 24h, 48h, and 72h (different shades of grey). The x-axis indicates the treatment; the y-axis indicates the maximum Caspase 3/7 induction (% of cells) seen for each treatment.

## DETAILED DESCRIPTION

In a first aspect, provided herein is a pharmaceutical combination comprising:

(a) a first compound having the structure of formula (I):

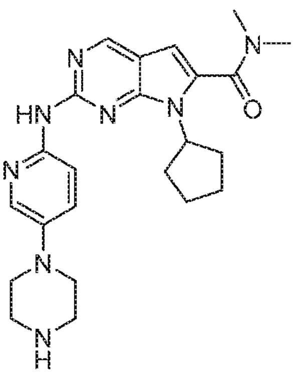

(I)

or a pharmaceutically acceptable salt or solvate thereof, and

(b) a second compound having the structure of formula (II):

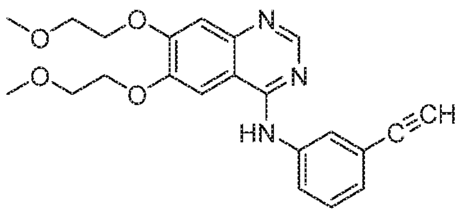

(II)

or a pharmaceutically acceptable salt or solvate thereof.

In an embodiment, the compound having the structure of formula (I), or a pharmaceutically acceptable salt or solvate thereof, and the compound having the structure of formula (II), or a pharmaceutically acceptable salt or solvate thereof, are in the same formulation.

In an embodiment, the compound having the structure of formula (I), or a pharmaceutically acceptable salt or solvate thereof, and the compound having the structure of formula (II), or a pharmaceutically acceptable salt or solvate thereof, are in separate formulations.

In an embodiment, the combination of the first aspect is for simultaneous or sequential administration.

In a particular embodiment of the pharmaceutical combinations described *supra,* the first compound is the succinate salt of the compound having the structure of formula (I).

In a second aspect, provided herein is a method for the treatment or prevention of cancer in a subject in need thereof, comprising administering to the subject a therapeutically effective amount of a pharmaceutical combination according to any one of the embodiments described *supra.*

In an embodiment, the cancer is selected from the group consisting of melanoma, lung cancer (including non-small-cell lung cancer (NSCLC)), colorectal cancer (CRC), breast cancer, kidney cancer, renal cell carcinoma (RCC), liver cancer, acute myelogenous leukemia (AML), myelodysplastic syndromes (MDS), thyroid cancer, pancreatic cancer, neurofibromatosis and hepatocellular carcinoma.

In a particular embodiment, the cancer is colorectal cancer.

In a third aspect, provided herein is a pharmaceutical combination as described *supra* for use in the treatment or prevention of cancer.

In a fourth aspect, provided herein is a pharmaceutical combination as described *supra* for use in the manufacture of a medicament for the treatment or prevention of cancer.

In certain embodiments of the third and fourth aspects, the cancer is selected from the group consisting of melanoma, lung cancer (including non-small-cell lung cancer (NSCLC)), colorectal cancer (CRC), breast cancer, kidney cancer, renal cell carcinoma (RCC), liver cancer, acute myelogenous leukemia (AML), myelodysplastic syndromes (MDS), thyroid cancer, pancreatic cancer, neurofibromatosis and hepatocellular carcinoma.

In a particular embodiment, the cancer is colorectal cancer.

In a fifth aspect, provided herein is the use of a pharmaceutical combination as described *supra* for the manufacture of a medicament for the treatment or prevention of cancer.

In a sixth aspect, provided herein is the use of a pharmaceutical combination as described *supra* for the treatment or prevention of cancer.

In particular embodiments of the fifth and sixth aspects, the cancer is selected from the group consisting of melanoma, lung cancer (including non-small-cell lung cancer (NSCLC)), colorectal cancer (CRC), breast cancer, kidney cancer, renal cell carcinoma (RCC), liver cancer, acute myelogenous leukemia (AML), myelodysplastic syndromes (MDS), thyroid cancer, pancreatic cancer, neurofibromatosis and hepatocellular carcinoma.

In a particular embodiment, the cancer is colorectal cancer.

In a seventh aspect, provided herein is a pharmaceutical composition comprising:

(a) a first compound having the structure of formula (I):

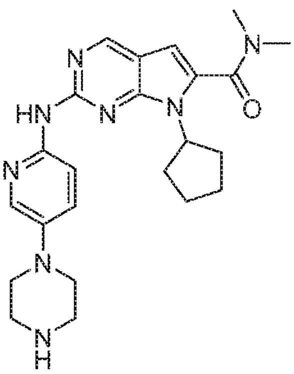

(I)

or a pharmaceutically acceptable salt or solvate thereof, and

(b) a second compound having the structure of formula (II):

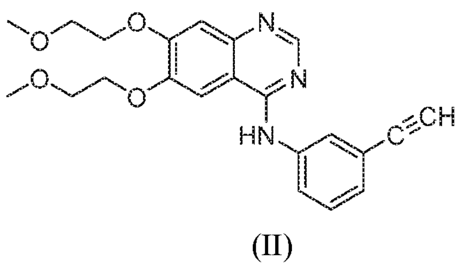

(II)

or a pharmaceutically acceptable salt or solvate thereof.

In an embodiment, the pharmaceutical composition comprises one or more excipients.

Inhibitor Compounds

The CDK 4/6 inhibitor 7-Cyclopentyl-2-(5-piperazin-1-yl-pyridin-2-ylamino)-7H-pyrrolo[2,3-d]pyrimidine-6-carboxylic acid dimethylamide (also known as "LEE011" or "ribociclib") is referred to herein as the compound having the

structure of formula (I), or compound (I):

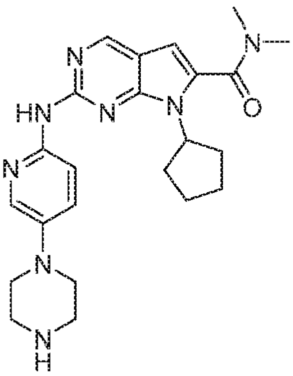

(I)

Compound (I), and pharmaceutically acceptable salts and solvates thereof are described in International Publication No. WO 2010/020675 (e.g., in Example 74).

The EGFR inhibitor N-(3-ethynylphenyl)-6,7-bis(2-methoxyethoxy)quinazolin-4-amine (known also as "erlotinib") is referred to herein as the compound having the structure of formula (II), or compound (II):

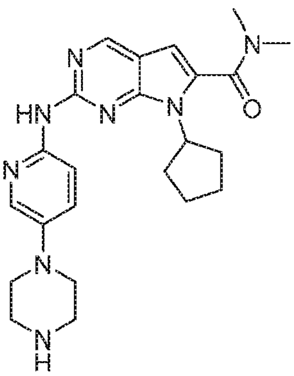

(II)

Compound (II), and pharmaceutically acceptable salts and solvates thereof are described in International Publication No. WO 96/30347 (e.g., Example 20).

Salts and Solvates

Salts of the inhibitor compounds described herein can be present alone or in a mixture with the free base form, and are preferably pharmaceutically acceptable salts. A "pharmaceutically acceptable salt", as used herein, unless otherwise indicated, includes salts of acidic and basic groups which may be present in the compounds of the present invention. Such salts may be formed, for example, as acid addition salts, preferably with organic or inorganic acids, upon reaction with a basic nitrogen atom. Suitable inorganic acids are, for example, halogen acids, such as hydrochloric acid, sulfuric acid, or phosphoric acid. Suitable organic acids are, e.g., carboxylic acids or sulfonic acids, such as fumaric acid or methansulfonic acid. For isolation or purification purposes it is also possible to use pharmaceutically unacceptable salts, for example picrates or perchlorates.

In a preferred embodiment of the pharmaceutical combinations described herein, the compound having the structure of formula (I) is in the form of a succinate salt.

For therapeutic use, only pharmaceutically acceptable salts, solvates or free compounds are employed (where applicable in the form of pharmaceutical preparations), and these are therefore preferred. In view of the close relationship between the compounds in their free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient. Salts contemplated herein are preferably pharmaceutically acceptable salts; suitable counter-ions forming

pharmaceutically acceptable salts are known in the field.

Pharmaceutical Combinations and Compositions

The combinations and compositions can be administered to a system comprising cells or tissues, as well as a human subject (e.g., a patient) or an animal subject.

The combination and composition of the present invention can be administered in various dosage forms and strength, in a pharmaceutically effective amount or a clinically effective amount.

The pharmaceutical compositions for separate administration of both combination components, or for the administration in a fixed combination, e.g., a single galenical composition comprising the combination, may be prepared in any manner known in the art and are those suitable for enteral, such as oral or rectal, and parenteral administration to mammals (warm-blooded animals), including humans.

The pharmaceutical compositions described herein may contain, from about 0.1 % to about 99.9%, preferably from about 1 % to about 60 %, of the therapeutic agent(s). Suitable pharmaceutical compositions for the combination therapy for enteral or parenteral administration are, for example, those in unit dosage forms, such as sugar-coated tablets, tablets, capsules or suppositories, or ampoules. If not indicated otherwise, these are prepared in a manner known *per se,* for example by means of various conventional mixing, comminution, direct compression, granulating, sugar-coating, dissolving, lyophilizing processes, or fabrication techniques readily apparent to those skilled in the art. It will be appreciated that the unit content of a combination partner contained in an individual dose of each dosage form need not in itself constitute an effective amount since the necessary effective amount may be reached by administration of a plurality of dosage units.

A unit dosage form containing the combination of agents or individual agents of the combination of agents may be in the form of micro-tablets enclosed inside a capsule, e.g., a gelatin capsule. For this, a gelatin capsule as is employed in pharmaceutical formulations can be used, such as the hard gelatin capsule known as CAPSUGEL, available from Pfizer.

The unit dosage forms of the present invention may optionally further comprise additional conventional carriers or excipients used for pharmaceuticals. Examples of such carriers include, but are not limited to, disintegrants, binders, lubricants, glidants, stabilizers, and fillers, diluents, colorants, flavours and preservatives. One of ordinary skill in the art may select one or more of the aforementioned carriers with respect to the particular desired properties of the dosage form by routine experimentation and without any undue burden. The amount of each carriers used may vary within ranges conventional in the art. The following references disclose techniques and excipients used to formulate oral dosage forms. See The Handbook of Pharmaceutical Excipients, 4th edition, Rowe et al., Eds., American Pharmaceuticals Association (2003); and Remington: the Science and Practice of Pharmacy, 20th edition, Gennaro, Ed., Lippincott Williams & Wilkins (2003).

As used herein, the term "pharmaceutically acceptable excipient" or "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

These optional additional conventional carriers may be incorporated into the oral dosage form either by incorporating the one or more conventional carriers into the initial mixture before or during granulation or by combining the one or more conventional carriers with granules comprising the combination of agents or individual agents of the combination of agents in the oral dosage form. In the latter embodiment, the combined mixture may be further blended, e.g., through a V-blender, and subsequently compressed or molded into a tablet, for example a monolithic tablet, encapsulated by a capsule, or filled into a sachet.

Examples of pharmaceutically acceptable disintegrants include, but are not limited to, starches; clays; celluloses; alginates; gums; cross-linked polymers, e.g., cross-linked polyvinyl pyrrolidone or crospovidone, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; soy polysaccharides; and guar gum. The disintegrant may be present in an amount from about 0% to about 10% by weight of the composition. In one embodiment, the disintegrant is present in an amount from about 0.1% to about 5% by weight of composition.

Examples of pharmaceutically acceptable binders include, but are not limited to, starches; celluloses and derivatives thereof, for example, microcrystalline cellulose, e.g., AVICEL PH from FMC (Philadelphia, PA), hydroxypropyl cellulose hydroxylethyl cellulose and hydroxylpropylmethyl cellulose METHOCEL from Dow Chemical Corp. (Midland, MI); sucrose; dextrose; corn syrup; polysaccharides; and gelatin. The binder may be present in an amount from about 0% to about 50%, e.g., 2-20% by weight of the composition.

Examples of pharmaceutically acceptable lubricants and pharmaceutically acceptable glidants include, but are

not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose and microcrystalline cellulose. The lubricant may be present in an amount from about 0% to about 10% by weight of the composition. In one embodiment, the lubricant may be present in an amount from about 0.1% to about 1.5% by weight of composition. The glidant may be present in an amount from about 0.1% to about 10% by weight.

Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from about 0% to about 80% by weight of the composition.

The optimal dosage of each combination partner for treatment of cancer can be determined empirically for each individual using known methods and will depend upon a variety of factors, including, though not limited to, the degree of advancement of the disease; the age, body weight, general health, gender and diet of the individual; the time and route of administration; and other medications the individual is taking. Optimal dosages may be established using routine testing and procedures that are well known in the art.

The amount of each combination partner that may be combined with the carrier materials to produce a single dosage form will vary depending upon the individual treated and the particular mode of administration. In some embodiments the unit dosage forms containing the combination of agents as described herein will contain the amounts of each agent of the combination that are typically administered when the agents are administered alone.

The effective dosage of each of the combination partners employed in the combination of the invention may vary depending on the particular compound or pharmaceutical composition employed, the mode of administration, the condition being treated, and the severity of the condition being treated. Thus, the dosage regimen of the combinations described herein are selected in accordance with a variety of factors including the route of administration and the renal and hepatic function of the patient.

The effective dosage of each of the combination partners may require more frequent administration of one of the compound(s) as compared to the other compound(s) in the combination. Therefore, to permit appropriate dosing, packaged pharmaceutical products may contain one or more dosage forms that contain the combination of compounds, and one or more dosage forms that contain one of the combination of compounds, but not the other compound(s) of the combination.

Compound (I) ("LEE011") (based on weight of the unsalted/unsolvated compound), in general, is administered in a dose in the range from 10 mg to 2000 mg per day in human. In one embodiment, LEE011 is administered 600mg QD. In another embodiment, LEE011 is administered 300mg QD. In another embodiment, LEE011 is administered in 900mg QD.

Compound (II) (erlotinib), in general, is administered in a dose in the range from 10 mg to 300 mg. In one embodiment, erolotinib is administered 100mg QD. In another embodiment, erlotinib is administered 150mg QD.

In all formulations, the active ingredient(s) forming part of a combination product according to the present invention can be present each in a relative amount of 0.5 to 95 % of weight of the corresponding formulation (regarding the formulation as such, that is without packaging and leaflet), e.g. from 1 to 90,5 to 95, 10 to 98 or 10 to 60 or 40 to 80 % by weight, respectively.

The dosage of the active ingredient to be applied to a warm-blooded animal depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy without toxicity requires a regimen based on the kinetics of the drug's availability to target sites. This involves a consideration of the distribution, equilibrium, and elimination of a drug. A pharmaceutical combination as described herein can be e.g., in unit dosage of about 1-1000 mg of active ingredient(s) for a subject of about 50-70 kg, or about 1-500 mg or about 1-250 mg or about 1-150 mg or about 0.5-100 mg, or about 1-50 mg of for any one or in particular the sum of active ingredients; or (especially for the EGFR inhibitor) 50 to 900, 60 to 850, 75 to 800 or 100 to 600 mg, respectively, for any one or in particular the sum of active ingredients. The therapeutically effective dosage of a compound, the pharmaceutical composition, or the combinations thereof, is dependent on the species of the subject, the body weight, age and individual condition, the disorder or disease or the severity thereof being treated. A physician, clinician or (in animal use) veterinarian of ordinary skill can readily determine the effective amount of each of the active ingredients necessary to prevent, treat or inhibit the progress of the disorder or disease.

The optimum ratios, individual and combined dosages, and concentrations of the combination partners of the combination of the invention (i.e., compound (I) and compound (II)) that yield efficacy without toxicity are based on the kinetics of the therapeutic agents' availability to target sites, and are determined using methods known to those of skill in the art.

Frequency of dosage may vary depending on the compound used and the particular condition to be treated or

prevented. In general, the use of the minimum dosage that is sufficient to provide effective therapy is preferred. Patients may generally be monitored for therapeutic effectiveness using assays suitable for the condition being treated or prevented, which will be familiar to those of ordinary skill in the art.

In certain aspects, the pharmaceutical combinations described herein are useful for the treatment or prevention of cancer, or for the preparation of a medicament for the treatment or prevention of cancer. In a particular embodiment, the pharmaceutical combinations described herein are useful for the treatment of cancer, or for the preparation of a medicament for the treatment of cancer.

In certain aspects, a method for the treatment or prevention of cancer (e.g., for the treatment of cancer) is provided, comprising administering to a patient in need thereof a pharmaceutically effective amount of a pharmaceutical combination described herein.

The nature of cancer is multifactorial. Under certain circumstances, drugs with different mechanisms of action may be combined. However, just considering any combination of therapeutic agents having different mode of action does not necessarily lead to combinations with advantageous effects.

The administration of a pharmaceutical combination as described herein may result not only in a beneficial effect, e.g., a synergistic therapeutic effect, e.g., with regard to alleviating, delaying progression of or inhibiting the symptoms, but also in further surprising beneficial effects, e.g., fewer side-effects, a more durable response, an improved quality of life or a decreased morbidity, compared with a monotherapy applying only one of the pharmaceutically therapeutic agents used in the combination of the invention.

A further benefit is that lower doses of the therapeutic agents of a pharmaceutical combination as described herein can be used, for example, such that the dosages may not only often be smaller, but are also may be applied less frequently, or can be used in order to diminish the incidence of side-effects observed with one of the combination partners alone. This is in accordance with the desires and requirements of the patients to be treated.

It can be shown by established test models that a pharmaceutical combination as described herein results in the beneficial effects described herein before. The person skilled in the art is fully enabled to select a relevant test model to prove such beneficial effects. The pharmacological activity of a combination of the invention may, for example, be demonstrated in a clinical study or in an animal model.

Determining a synergistic interaction between one or more components, the optimum range for the effect and absolute dose ranges of each component for the effect may be definitively measured by administration of the components over different w/w ratio ranges and doses to patients in need of treatment. For humans, the complexity and cost of carrying out clinical studies on patients may render impractical the use of this form of testing as a primary model for synergy. However, the observation of synergy in certain experiments (see, e.g., example 1) can be predictive of the effect in other species and animal models exist to further measure a synergistic effect. The results of such studies can also be used to predict effective dose ratio ranges and the absolute doses and plasma concentrations.

In an embodiment, the combinations and/or compositions provided herein display a synergistic effect.

In an embodiment, provided herein is a synergistic combination for administration to a human, said combination comprising the inhibitors described herein, where the dose range of each inhibitor corresponds to the synergistic ranges suggested in a suitable tumor model or clinical study.

When the combination partners, which are employed in the combination of the invention, are applied in the form as marketed as single drugs, their dosage and mode of administration can be in accordance with the information provided on the package insert of the respective marketed drug, if not mentioned herein otherwise.

Definitions

Certain terms used herein are described below. Compounds are described using standard nomenclature. Unless defined otherwise, all technical and scientific terms used herein have the meaning that is commonly understood by one of skill in the art to which the present disclosure belongs.

The term "pharmaceutical composition" is defined herein to refer to a mixture or solution containing at least one therapeutic agent to be administered to a subject, e.g., a mammal or human, in order to prevent or treat a particular disease or condition affecting the mammal or human.

The term "pharmaceutically acceptable" is defined herein to refer to those compounds, materials, compositions and/or dosage forms, which are, within the scope of sound medical judgment, suitable for contact with the tissues a subject, e.g., a mammal or human, without excessive toxicity, irritation allergic response and other problem complications commensurate with a reasonable benefit / risk ratio.

The term "treating" or "treatment" as used herein comprises a treatment relieving, reducing or alleviating at least one symptom in a subject or effecting a delay of progression of a disease. For example, treatment can be the diminishment of one or several symptoms of a disorder or complete eradication of a disorder, such as cancer. Within the meaning of the present invention, the term "treat" also denotes to arrest, delay the onset (i.e., the period prior to clinical manifestation of a disease) and/or reduce the risk of developing or worsening a disease. The term "prevent",

"preventing" or "prevention" as used herein comprises the prevention of at least one symptom associated with or caused by the state, disease or disorder being prevented.

The term "pharmaceutically effective amount" or "clinically effective amount" of a combination of therapeutic agents is an amount sufficient to provide an observable improvement over the baseline clinically observable signs and symptoms of the disorder treated with the combination.

The term "combination," "therapeutic combination," or "pharmaceutical combination" as used herein refer to either a fixed combination in one dosage unit form, or non-fixed combination or a kit of parts for the combined administration where two or more therapeutic agents may be administered independently, at the same time, or separately within time intervals, especially where these time intervals allow that the combination partners to show a cooperative, e.g., synergistic, effect.

The term "combination therapy" refers to the administration of two or more therapeutic agents to treat a therapeutic condition or disorder described in the present disclosure. Such administration encompasses co-administration of these therapeutic agents in a substantially simultaneous manner, such as in a single formulation having a fixed ratio of active ingredients or in separate formulations (*e.g.,* capsules and/or intravenous formulations) for each active ingredient. In addition, such administration also encompasses use of each type of therapeutic agent in a sequential or separate manner, either at approximately the same time or at different times. Regardless of whether the active ingredients are administered as a single formulation or in separate formulations, the therapeutic agents are administered to the same patient as part of the same course of therapy. In any case, the treatment regimen will provide beneficial effects in treating the conditions or disorders described herein.

The term "synergistic effect" as used herein refers to action of two therapeutic agents such as, for example, the CDK inhibitor LEE011, and the EGFR inhibitor Erlotinib, producing an effect, for example, slowing the symptomatic progression of a proliferative disease, particularly cancer, or symptoms thereof, which is greater than the simple addition of the effects of each therapeutic agent administered alone. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively.

The term "subject" or "patient" as used herein includes animals, which are capable of suffering from or afflicted with a cancer or any disorder involving, directly or indirectly, a cancer. Examples of subjects include mammals, e.g., humans, dogs, cows, horses, pigs, sheep, goats, cats, mice, rabbits, rats and transgenic non-human animals. In the preferred embodiment, the subject is a human, e.g., a human suffering from, at risk of suffering from, or potentially capable of suffering from cancer.

The terms "fixed combination" and "fixed dose" and "single formulation" as used herein refer to single carrier or vehicle or dosage forms formulated to deliver an amount, which is jointly therapeutically effective for the treatment of cancer, of two or more therapeutic agents to a patient. The single vehicle is designed to deliver an amount of each of the agents, along with any pharmaceutically acceptable carriers or excipients. In some embodiments, the vehicle is a tablet, capsule, pill, or a patch. In other embodiments, the vehicle is a solution or a suspension.

The term "non-fixed combination," "kit of parts," and "separate formulations" means that the active ingredients, e.g., LEE011 and Erlotinib are both administered to a patient as separate entities either simultaneously, concurrently or sequentially with no specific time limits, wherein such administration provides therapeutically effective levels of the two compounds in the body of the warm-blooded animal in need thereof. The latter also applies to cocktail therapy, e.g., the administration of three or more active ingredients.

The term "unit dose" is used herein to mean simultaneous administration of two or three agents together, in one dosage form, to the patient being treated. In some embodiments, the unit dose is a single formulation. In certain embodiments, the unit dose includes one or more vehicles such that each vehicle includes an effective amount of at least one of the agents along with pharmaceutically acceptable carriers and excipients. In some embodiments, the unit dose is one or more tablets, capsules, pills, injections, infusions, patches, or the like, administered to the patient at the same time.

An "oral dosage form" includes a unit dosage form prescribed or intended for oral administration.

The terms "comprising" and "including" are used herein in their open-ended and nonlimiting sense unless otherwise noted.

The terms "a" and "an" and "the" and similar references in the context of describing the invention (especially in the context of the following claims) are to be construed to cover both the singular and the plural, unless otherwise indicated herein or clearly contradicted by context. Where the plural form is used for compounds, salts, and the like, this is taken to mean also a single compound, salt, or the like.

The term "about" or "approximately" shall have the meaning of within 10%, more preferably within 5%, of a

given value or range.

EXAMPLES

Materials and Methods

The compounds were dissolved in 100% DMSO (Sigma, Catalog number D2650) at concentrations of 20 mM and stored at -20°C until use. Compounds were arrayed in drug master plates (Greiner, Catalog number 788876) and serially diluted 3-fold (7 steps) at 2000X concentration.

Colorectal cancer cell lines used for this study were obtained, cultured and processed from commercial vendors ATCC, CellBank Australia, DMSZ, ECACC, and HSRRB (Table 1). All cell line media were supplemented with 10% FBS (HyClone, Catalog number SH30071.03). Media for LIM2551 was additionally supplemented with 0.6 μg/mL Insulin (SIGMA, Catalog number 19278), 1 μg/mL Hydrocortisone (SIGMA, Catalog number H0135), and 10 μM 1-Thioglycerol (SIGMA, Catalog number M6145).

| Cell line | Driver mutations | Source | Source Cat Num | Medium | Medium Vendor | Medium Cat Num | #Cells | Treatment [h] |
|---|---|---|---|---|---|---|---|---|
| DLD-1 | KRAS, PIK3CA | ATCC | CCL-221 | RPMI | ThermoFisher | 22400-071 | 500 | 72 |
| HCT-116 | KRAS, PIK3CA | ATCC | CCL-247 | McCoy's 5A | ATCC | 30-2007 | 500 | 72 |
| LS-180 | KRAS, PIK3CA | ATCC | CCL-187 | EMEM | ATCC | 30-2003 | 800 | 72 |
| GP2d | KRAS, PIK3CA | ECACC | 95090714 | DMEM | ATCC | 30-2002 | 900 | 72 |
| SW480 | KRAS | ATCC | CCL-228 | RPMI | ATCC | 30-2001 | 700 | 72 |
| SW837 | KRAS | ATCC | CCL-235 | RPMI | ATCC | 30-2001 | 1250 | 72 |
| LoVo | KRAS | ATCC | CCL-229 | F-12K | ATCC | 30-2004 | 1250 | 96 |
| RKO | BRAF,PIK3CA | ATCC | CRL-2577 | EMEM | ATCC | 30-2003 | 500 | 72 |
| LIM2551 | BRAF,PIK3CA | CellBank Australia | CBA-0170 | RPMI | ATCC | 30-2001 | 1000 | 72 |
| HT-29 | BRAF,PIK3CA | ATCC | HTB-38 | McCoy's 5A | ATCC | 30-2007 | 800 | 72 |
| OUMS-23 | BRAF | HSRRB | JCRB1022 | DMEM | ATCC | 30-2002 | 900 | 72 |
| LS411N | BRAF | ATCC | CRL-2159 | RPMI | ATCC | 30-2001 | 900 | 72 |
| COLO-205 | BRAF | ATCC | CCL-222 | RPMI | ATCC | 30-2001 | 800 | 72 |
| NCI-H508 | PIK3CA | ATCC | CCL-253 | RPMI | ATCC | 30-2001 | 1000 | 72 |
| COLO-320 | | DSMZ | ACC-144 | RPMI | ATCC | 30-2001 | 800 | 72 |

**Table 1.** Cell line information

Cell lines were cultured in 37 °C and 5% $CO_2$ incubator and expanded in T-75 flasks. In all cases cells were thawed from frozen stocks, expanded through ≥1 passage using 1:3 dilutions, counted and assessed for viability using a ViCell counter (Beckman-Coulter) prior to plating. To split and expand cell lines, cells were dislodged from flasks using 0.25% Trypsin-EDTA (GIBCO, Catalog number 25200). All cell lines were determined to be free of mycoplasma contamination as determined by a PCR detection methodology performed at Idexx Radii (Columbia, MO, USA) and correctly identified by detection of a panel of SNPs.

Images were analyzed after adapting previously described methods (Horn, Sandmann et al. 2011) and using the Bioconductor package EBImage in R (Pau, Fuchs et al. 2010). Objects in both channels, DAPI (for Hoechst/DNA) and FITC (for Caspase 3/7), were segmented separately by adaptive thresholding and counted. A threshold for Caspase 3/7 positive objects was defined manually per cell line after comparing negative controls (DMSO) and positive controls (Staurosporine). By analyzing 17 additional object/nuclei features in the DNA channel (shape and intensity features) debris/fragmented nuclei were identified. To this end per cell line the distributions of the additional features between positive controls (Staurosporine) and negative controls (DMSO) were compared manually. Features that could differentiate between the conditions (e.g. a shift in the distribution of a feature measurement comparing DMSO with Staurosporine) where used to define the 'debris' population versus the population of 'viable' nuclei. The debris counts were subtracted from raw nuclei counts. The resulting nuclei number was used as measure of cell proliferation ('cell count').

The compound's effect on cell proliferation was calculated from the cell counts of the treatments relative to the cell counts of the negative control (DMSO), in Figure 1 denoted as 'Normalized cell count' (= 'xnorm') on the y-axis. Synergistic combinations were identified using the highest single agent model (HSA) as null hypothesis (Berenbaum 1989). Excess over the HSA model predicts a functional connection between the inhibited targets (Lehar, Zimmermann et al. 2007, Lehar, Krueger et al. 2009). The model input were inhibition values per drug dose:

$$\mathbf{I = 1 - xnorm}$$

I: inhibition
xnorm: normalized cell count (median of three replicates)

At every dose point of the combination treatment the difference between the inhibition of the combination and the inhibition of the stronger of the two single agents was calculated (= model residuals). To favor combination effects at high inhibition the residuals were weighted with the observed inhibition at the same dose point. The overall combination score C of a drug combination is the sum of the weighted residuals over all concentrations:

$$\mathbf{C = \Sigma_{Conc} (I_{data} * (I_{data} - I_{model}))}$$

$I_{data}$: measured inhibition
$I_{model}$: inhibition according to HSA null hypothesis

Robust combination z-scores ($z_C$) were calculated as the ratio of the treatments' combination scores C and the median absolute deviation (mad) of non-interacting combinations:

$$\mathbf{z_C = C / mad(C_{zero})}$$

$C_{zero}$: combination scores of non-interacting combinations
$z_C$ is an indicator for the strength of the combination with:

$$z_C \geq 3\text{: synergy}$$

$$3 > z_C \geq 2\text{: weak synergy}$$

$$z_C < 2\text{: no synergy}$$

IC50 is the compound concentration that results in 50% of the cell counts relative to DMSO. IC50 calculations (see Table 2) were done using the DRC package in R (Ritz and Streibig 2005) and fitting a four-parameter log-logistic function to the data.

The compound's effect on apoptosis was determined by calculating the percentage of cells with activated Caspase 3/7 per treatment and time point relative to the raw cell counts (before subtraction of debris) (y-axis in Figure 2). Cell counts at time points that were not experimentally measured were obtained by regression analysis by fitting a linear model for log-transformed cell counts at day 0 and the end of the treatment (assuming exponential cell growth).

**EXAMPLE 1: The *in vitro* effect on proliferation of combining the CDK4/6 inhibitor LEE011 with the EGFR inhibitor erlotinib in colorectal cancer cell lines.**

To test the effect of the combination of LEE011 and erlotinib on cell proliferation cells were plated in black 384-well microplates with clear bottom (Matrix/Thermo Scientific, Catalog number 4332) in 50 μL media per well at cell densities between 500 and 1250 cells/well (Table 1) and allowed to incubate at 37 degrees, 5% $CO_2$ for 24h. After 24h one 384-well plate per cell line was prepared for cell counting by microscopy (see below) without receiving treatment (= 'baseline'). The other cell plates were treated by transferring 25 nL of the 2000X compound from drug master plates using an ATS acoustic liquid dispenser (ECD Biosystems) and resulting in a final 1X concentration. LEE011 was used over a final concentration range of 13 nM - 10 μM, and erlotinib was used over a final concentration range of 13 nM - 10 μM (71:3 dilution steps). For the combination of LEE011 with erlotinib the single agents were combined at a fixed ratio of 1:1 at each dilution resulting in 7 combination treatments. Additionally, negative controls (DMSO = 'vehicle') and positive controls (Staurosporine = killing cells, 7-point 1:2 dilution series for a dose range of 16 nM - 1 μM) were transferred as treatment controls, and compounds with no efficacy in the cell lines tested were used in combinations with LEE011 and erlotinib as combination controls (combinations that do not exceed the efficacy of the more efficacious single agent = 'non-interacting' combinations). After compound addition 50 nL of 2 mM CellEvent Caspase-3/7 Green Detection Reagent (ThermoFisher, Catalog number C10423) were added to one of the three replicates using the HP D300 Digital Dispenser (Tecan). Caspase 3/7 induction was measured as a proxy for apoptosis induced by the treatments. Cells were treated for 72h to 96h depending on their doubling time (Table 1), and Caspase 3/7 activation was measured every 24h by microscopy using an InCell Analyzer 2000 (GE Healthcare) equipped with a 4X objective and FITC excitation/emission filters. At the end of the treatment cells were prepared for cell counting by microscopy. Cells were fixed and permeabilised for 45 minutes in 4% PFA (Electron Microscopy Sciences, Catalog number 15714), 0.12% TX-100 (Electron Microscopy Sciences, Catalog number 22140) in PBS (Boston Bioproducts, Catalog number BM-220). After washing cells three times with PBS their DNA was stained for 30 minutes with Hoechst 33342 (ThermoFisher, Catalog number H3570) at a final concentration of 4 μg/mL. Cells were washed three times with PBS and then plates were heat-sealed using a PlateLoc (Agilent Technologies) with aluminum seals (Agilent Technologies, Catalog number 06644-001) and stored at 4°C until imaging. All cells per well/treatment were captured in a single image by fluorescence microscopy using an InCell Analyzer 2000 (GE Healthcare) equipped with a 4X objective and DAPI excitation/emission filters.

The efficacies of the CDK4/6 inhibitor LEE011 and the EGFR inhibitor erlotinib were assessed individually and in combination in a total of 15 colorectal cancer cell lines. Cell lines were mutant in KRAS, BRAF, and/or PIK3CA, or wild type for all 3 genes (Table 1). LEE011 as single agent inhibited the growth of all but two cell lines (SW837, OUMS-23) with sub-micromolar to micromolar IC50 values (Figure 1 and Table 2). Erlotinib as single agent only achieved IC50s for the concentration range tested in 5/15 cell lines (Figure 1 and Table 2). The combination treatment caused synergistic inhibition (according to the HSA model) in 7/15 lines tested, and weak synergistic inhibition in 1/15 lines (Table 2). Synergies were significantly stronger in KRAS mutant models compared to BRAF mutant models (p=0.005, one-tailed t-test), and the inhibitions and synergies were also significantly stronger in KRAS/BRAF wild type models compared to BRAF mutant models (p=0.05 and $p=4*10^{-6}$, respectively, one-tailed t-test). The combination does not induce apoptosis (assessed by measuring Caspase 3/7 induction) (Figure 2). Combined inhibition of CDK4/6 and EGFR in colorectal cancer may provide an effective therapeutic modality capable of improving responses compared to each of the single agents and lead to more durable responses in the clinic.

| Cell | IC50 LEE011 | IC50 Erlotinib | Synergy z-score ($z_c$) |
|---|---|---|---|
| LoVo | 0.8 | 0.716 | 13.9 |
| GP2d | 4.5 | 8.861 | 9.6 |
| LS-180 | 1.8 | >10 | 7.6 |
| DLD-1 | 4.1 | 7.064 | 7 |
| COLO-320 | 2.8 | 6.92 | 4.3 |
| NCI-H508 | 0.7 | 0.>102 | 4.3 |
| SW837 | >10 | >10 | 3.7 |
| SW480 | 1.6 | >10 | 2.7 |
| HCT-116 | 4.5 | >10 | 1.2 |
| LIM2551 | 1.3 | >10 | 1.1 |
| COLO-205 | 1.1 | >10 | 1 |
| HT-29 | 0.8 | >10 | 0.6 |
| RKO | 1.5 | >10 | 0.3 |
| OUMS-23 | >10 | >10 | 0 |
| LS411N | 2.1 | >10 | -0.1 |

**Table 2.** Single agent IC50 values for each compound and synergy z-score measurements for the combination of LEE011 and erlotinib.

## Claims

1. A pharmaceutical combination comprising:

(a) a first compound having the structure of formula (I):

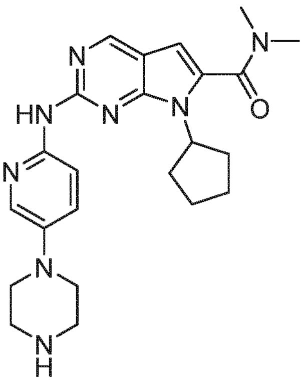

(I)

or a pharmaceutically acceptable salt or solvate thereof, and
(b) a second compound having the structure of formula (II):

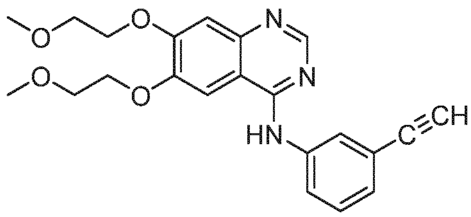

(II)

or a pharmaceutically acceptable salt or solvate thereof.

2. The pharmaceutical combination of claim 1, wherein the compound having the structure of formula (I) and the compound having the structure of formula (II) are in the same formulation.

3. The pharmaceutical combination of claim 1, wherein the compound having the structure of formula (I) and the compound having the structure of formula (II) are in separate formulations.

4. The pharmaceutical combination of claim 1, wherein the combination is for simultaneous or sequential administration.

5. The pharmaceutical combination of any one of claims 1-4, wherein the first compound is the succinate salt of formula (I).

6. The pharmaceutical combination of any one of claims 1-5, for use in the treatment of cancer.

7. The pharmaceutical combination of any one of claims 1-5, for use in the prevention of cancer.

8. The pharmaceutical combination for use according to claim 6 or 7 , wherein the cancer is selected from the group consisting of melanoma, lung cancer (including non-small-cell lung cancer (NSCLC)), colorectal cancer (CRC), breast cancer, kidney cancer, renal cell carcinoma (RCC), liver cancer, acute myelogenous leukemia (AML), myelodysplastic syndromes (MDS), thyroid cancer, pancreatic cancer, neurofibromatosis and hepatocellular carcinoma.

9. The pharmaceutical combination for use according to claim 8, wherein the cancer is colorectal cancer.

10. The pharmaceutical combination for use according to claim 9 wherein the colorectal cancer is KRAS-mutant and PIK3CA-mutant.

11. The pharmaceutical combination for use according to claim 9 wherein the colorectal cancer is KRAS-mutant.

**12.** The pharmaceutical combination for use according to claim 9 wherein the colorectal cancer is BRAF-mutant and PIK3CA-mutant.

**13.** The pharmaceutical combination for use according to claim 9 wherein the colorectal cancer is PIK3CA-mutant.

**14.** A pharmaceutical composition comprising:

(a) a first compound having the structure of formula (I):

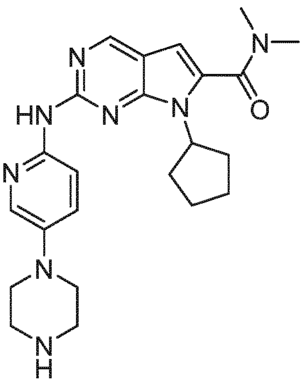

(I)

or a pharmaceutically acceptable salt or solvate thereof, and
(b) a second compound having the structure of formula (II):

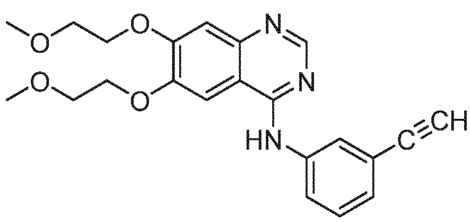

(II)

or a pharmaceutically acceptable salt or solvate thereof.

**15.** The pharmaceutical composition of claim 14, further comprising one or more excipients.

**Patentansprüche**

**1.** Pharmazeutische Kombination, umfassend:

(a) eine erste Verbindung mit der Struktur der Formel (I):

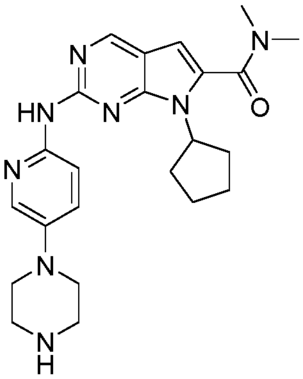

(I)

oder ein pharmazeutisch unbedenkliches Salz oder Solvat davon und
(b) eine zweite Verbindung mit der Struktur der Formel (II):

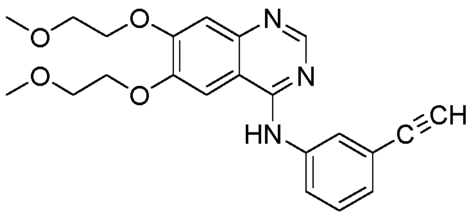

(II)

oder ein pharmazeutisch unbedenkliches Salz oder Solvat davon.

**2.** Pharmazeutische Kombination nach Anspruch 1, wobei die Verbindung mit der Struktur der Formel (I) und die Verbindung mit der Struktur der Formel (II) in der gleichen Formulierung vorliegen.

**3.** Pharmazeutische Kombination nach Anspruch 1, wobei die Verbindung mit der Struktur der Formel (I) und die Verbindung mit der Struktur der Formel (II) in getrennten Formulierungen vorliegen.

**4.** Pharmazeutische Kombination nach Anspruch 1, wobei die Kombination für eine gleichzeitig oder nacheinander erfolgende Verabreichung vorgesehen ist.

**5.** Pharmazeutische Kombination nach einem der Ansprüche 1-4, wobei es sich bei der ersten Verbindung um das Succinatsalz der Formel (I) handelt.

**6.** Pharmazeutische Kombination nach einem der Ansprüche 1-5, zur Verwendung bei der Behandlung einer Krebserkrankung.

**7.** Pharmazeutische Kombination nach einem der Ansprüche 1-5, zur Verwendung bei der Vorbeugung einer Krebserkrankung.

**8.** Pharmazeutische Kombination zur Verwendung gemäß Anspruch 6 oder 7, wobei die Krebserkrankung ausgewählt ist aus der Gruppe bestehend aus Melanom, Lungenkrebs (einschließlich nicht-kleinzelligem Lungenkrebs (Non-Small-Cell Lung Cancer, NSCLC)), Kolorektalkarzinom (CRC), Brustkrebs, Nierenkrebs, Nierenzellkarzinom (Renal Cell Carcinoma, RCC), Leberkrebs, akuter myeloischer Leukämie (AML), myelodysplastischen Syndromen (MDS), Schilddrüsenkrebs, Bauchspeicheldrüsenkrebs, Neurofibromatose und hepatozellulärem Karzinom.

**9.** Pharmazeutische Kombination zur Verwendung gemäß Anspruch 8, wobei es sich bei der Krebserkrankung um Kolorektalkarzinom handelt.

**10.** Pharmazeutische Kombination zur Verwendung gemäß Anspruch 9, wobei das Kolorektalkarzinom eine KRAS-Mutation und PIK3CA-Mutation aufweist.

**11.** Pharmazeutische Kombination zur Verwendung gemäß Anspruch 9, wobei das Kolorektalkarzinom eine KRAS-Mutation aufweist.

**12.** Pharmazeutische Kombination zur Verwendung gemäß Anspruch 9, wobei das Kolorektalkarzinom eine BRAF-Mutation und PIK3CA-Mutation aufweist.

**13.** Pharmazeutische Kombination zur Verwendung gemäß Anspruch 9, wobei das Kolorektalkarzinom eine PIK3CA-Mutation aufweist.

**14.** Pharmazeutische Zusammensetzung, umfassend:

(a) eine erste Verbindung mit der Struktur der Formel (I):

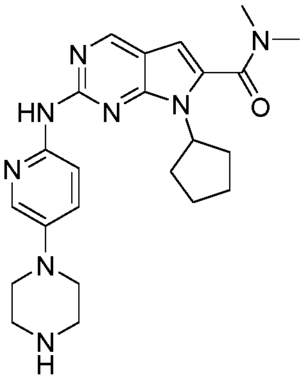

(I)

oder ein pharmazeutisch unbedenkliches Salz oder Solvat davon und
(b) eine zweite Verbindung mit der Struktur der Formel (II):

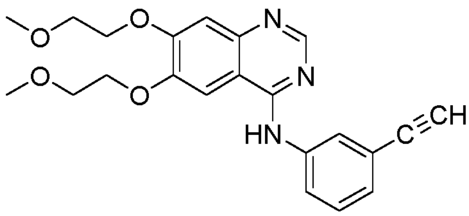

(II)

oder ein pharmazeutisch unbedenkliches Salz oder Solvat davon.

**15.** Pharmazeutische Zusammensetzung nach Anspruch 14, ferner umfassend einen oder mehrere Exzipienten.

## Revendications

**1.** Combinaison pharmaceutique comprenant :

(a) un premier composé possédant la structure de formule (I) :

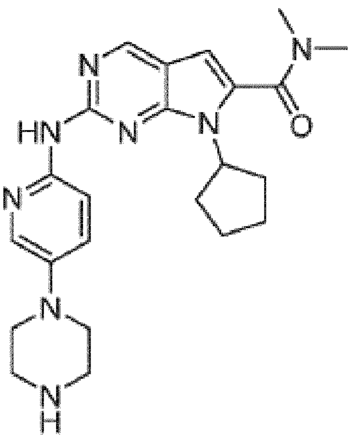

(I)

ou un sel ou solvate pharmaceutiquement acceptable de celui-ci, et
(b) un deuxième composé possédant la structure de formule (II) :

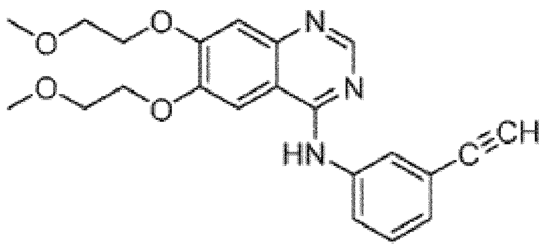

(II)

ou un sel ou solvate pharmaceutiquement acceptable de celui-ci.

2. Combinaison pharmaceutique selon la revendication 1, le composé possédant la structure de formule (I) et le composé possédant la structure de formule (II) se trouvant dans la même formulation.

3. Combinaison pharmaceutique selon la revendication 1, le composé possédant la structure de formule (I) et le composé possédant la structure de formule (II) se trouvant dans des formulations distinctes.

4. Combinaison pharmaceutique selon la revendication 1, la combinaison étant destinée à une administration simultanée ou séquentielle.

5. Combinaison pharmaceutique selon l'une quelconque des revendications 1 à 4, le premier composé étant le sel de succinate de formule (I).

6. Combinaison pharmaceutique selon l'une quelconque des revendications 1 à 5 pour une utilisation dans le traitement du cancer.

7. Combinaison pharmaceutique selon l'une quelconque des revendications 1 à 5 pour une utilisation dans la prévention du cancer.

8. Combinaison pharmaceutique pour une utilisation selon l'une quelconque des revendications 6 ou 7, le cancer étant choisi dans le groupe constitué par le mélanome, le cancer du poumon (y compris le cancer du poumon non à petites cellules (CPNPC)), le cancer colorectal (CRC), le cancer du sein, le cancer du rein, le carcinome à cellules rénales (CCR), le cancer du foie, la leucémie myéloïde aiguë (LMA), les syndromes myélodysplasiques (SMD), le cancer de la thyroïde, le cancer du pancréas, la neurofibromatose et le carcinome hépatocellulaire.

9. Combinaison pharmaceutique pour une utilisation selon la revendication 8, le cancer étant le cancer colorectal.

**10.** Combinaison pharmaceutique pour une utilisation selon la revendication 9, le cancer colorectal étant à mutation KRAS et à mutation PIK3CA.

**11.** Combinaison pharmaceutique pour une utilisation selon la revendication 9, le cancer colorectal étant à mutation KRAS.

**12.** Combinaison pharmaceutique pour une utilisation selon la revendication 9, le cancer colorectal étant à mutation BRAF et à mutation PIK3CA.

**13.** Combinaison pharmaceutique pour une utilisation selon la revendication 9, le cancer colorectal étant à mutation PIK3CA.

**14.** Composition pharmaceutique comprenant :

(a) un premier composé possédant la structure de formule (I) :

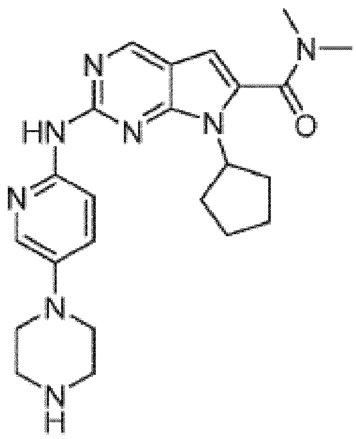

(I)

ou un sel ou solvate pharmaceutiquement acceptable de celui-ci, et
(b) un deuxième composé possédant la structure de formule (II) :

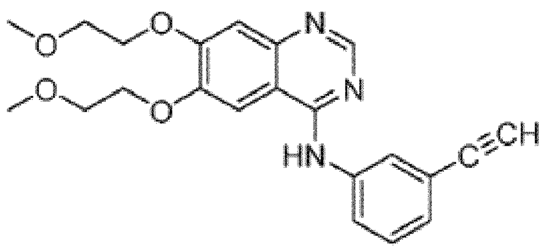

(II)

ou un sel ou solvate pharmaceutiquement acceptable de celui-ci.

**15.** Composition pharmaceutique selon la revendication 14, comprenant en outre un ou plusieurs excipient(s).

FIGURE 1

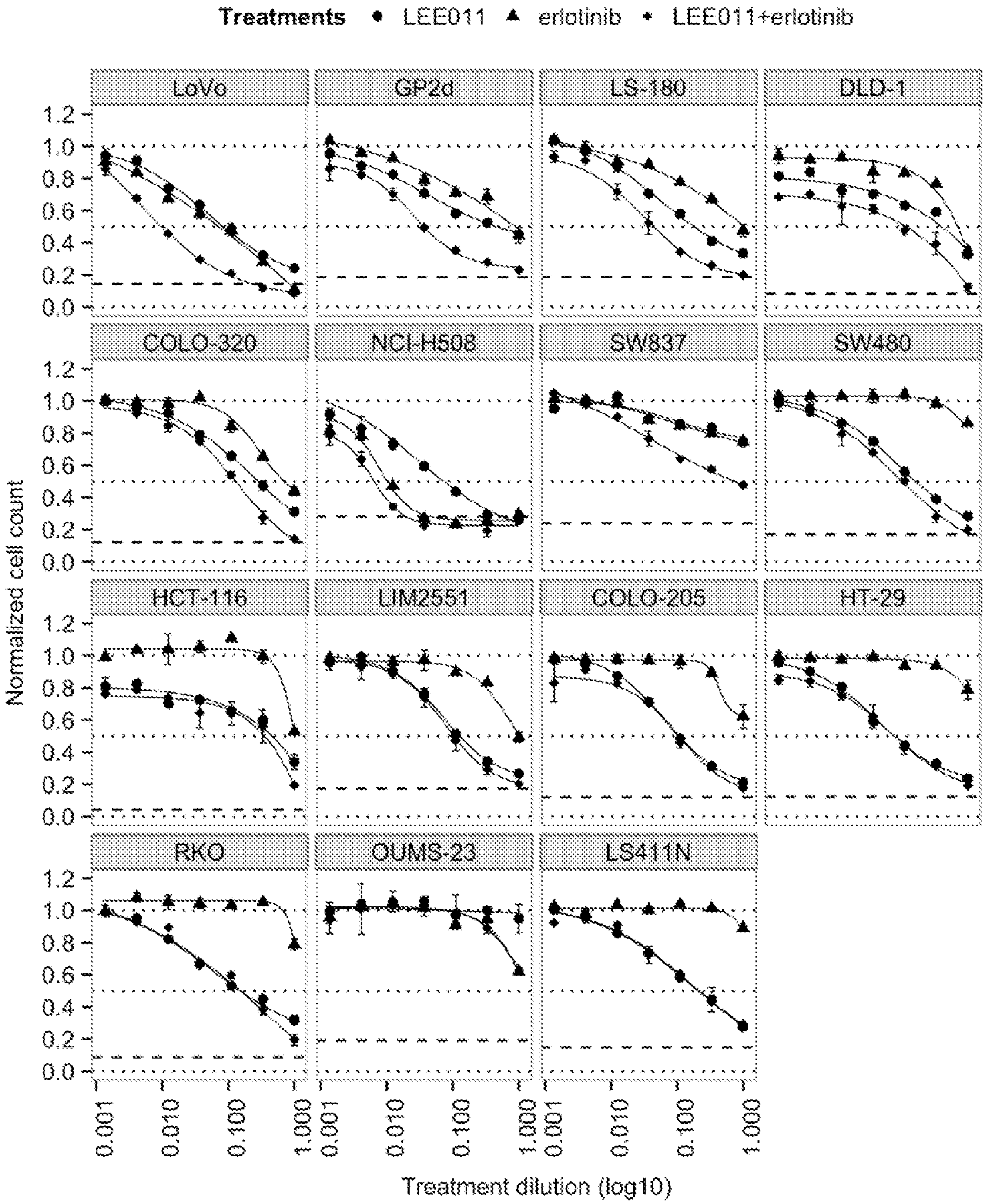
Treatments • LEE011 ▲ erlotinib • LEE011+erlotinib
LoVo
GP2d
LS-180
DLD-1
COLO-320
NCI-H508
SW837
SW480
HCT-116
LIM2551
COLO-205
HT-29
RKO
OUMS-23
LS411N
Normalized cell count
1.2
1.0
0.8
0.6
0.4
0.2
0.0
0.001
0.010
0.100
1.000
Treatment dilution (log10)

**FIGURE 2**

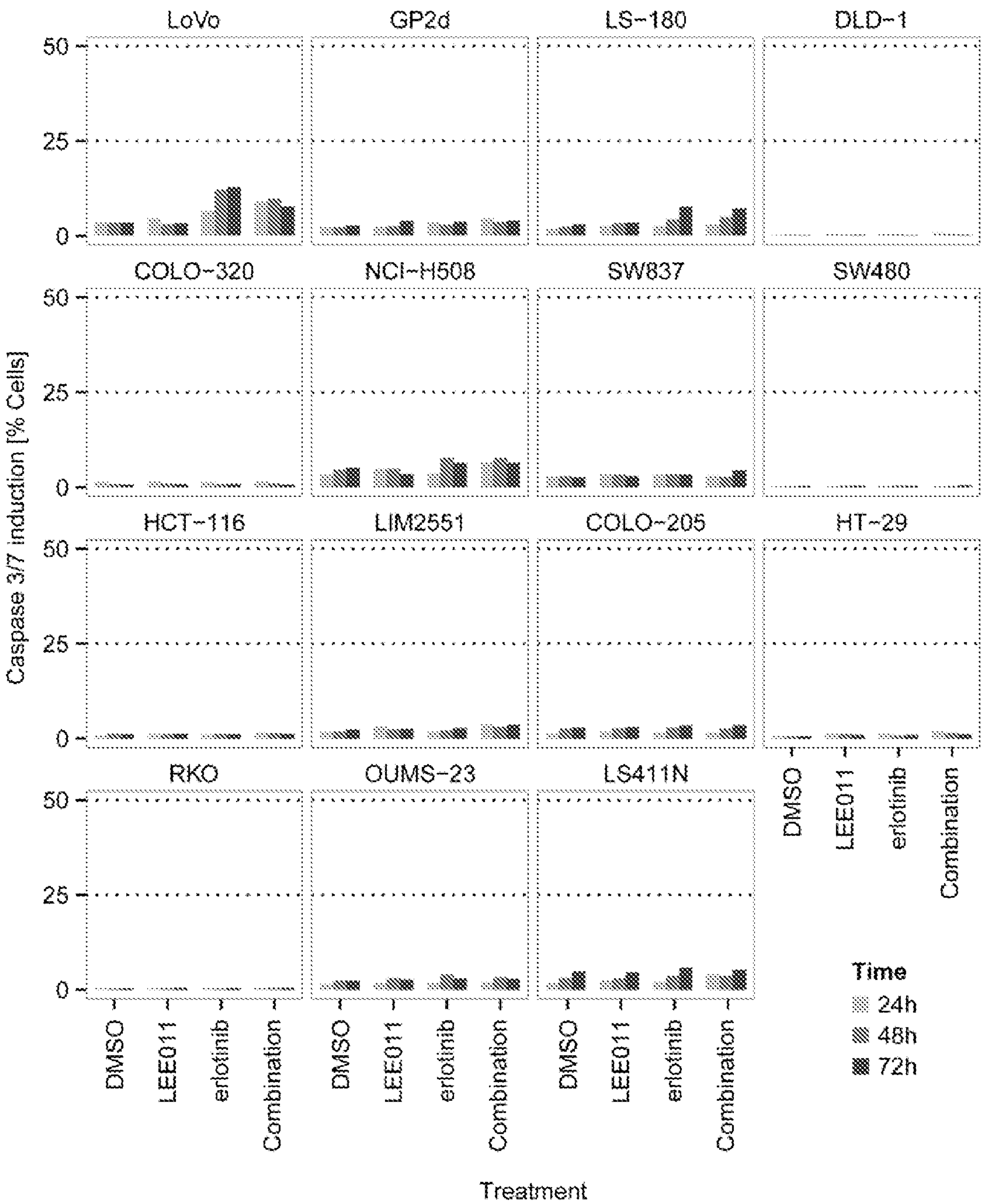
LoVo
GP2d
LS-180
DLD-1
COLO-320
NCI-H508
SW837
SW480
HCT-116
LIM2551
COLO-205
HT-29
RKO
OUMS-23
LS411N
50
25
0
Caspase 3/7 induction [% Cells]
DMSO
LEE011
erlotinib
Combination
Treatment
Time
24h
48h
72h

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010020675 A
- WO 9630347 A


### Non-patent literature cited in the description


- **FISCHER, P. M.** *Curr. Opin. Drug Discovery Dev.,* 2001, vol. 4, 623-634
- **LYNCH, T. J. et al.** *N Engl J Med,* 2004, vol. 350, 2129-2139
- **PAEZ, J. G. et al.** *Science,* 2004, vol. 304, 1497-1500
- **ENGELMAN, J. A. et al.** *Science,* 2007, vol. 316, 1039-1043
- **YANO, S. et al.** *Cancer Res,* 2008, vol. 68, 9479-9487
- **TURKE, A. B. et al.** *Cancer Cell,* 2010, vol. 17, 77-88
- **BACHLEITNER-HOFMANN, T. et al.** *Mol Cancer Ther,* 2008, vol. 7, 3499-3508
- The Handbook of Pharmaceutical Excipients. American Pharmaceuticals Association, 2003
- Remington: the Science and Practice of Pharmacy. Lippincott Williams & Wilkins, 2003
- Remington's Pharmaceutical Sciences. Mack Printing Company, 1990, 1289-1329
- **HOLFORD, N. H. G. ; SCHEINER, L. B.** *Clin. Pharmacokinet.,* 1981, vol. 6, 429-453
- **LOEWE, S. ; MUISCHNEK, H.** *Arch. Exp. Pathol Pharmacol.,* 1926, vol. 114, 313-326
- **CHOU, T. C. ; TALALAY, P.** *Adv. Enzyme Regul.,* 1984, vol. 22, 27-55